Europäisches Patentamt

European Patent Office

Office européen des brevets

(19)

(11) Veröffentlichungsnummer: **0 013 568**

**A1**

(12) # EUROPÄISCHE PATENTANMELDUNG

(21) Anmeldenummer: 80100088.6

(22) Anmeldetag: 09.01.80

(51) Int. Cl.³: **C 07 C 103/52**
**A 61 K 37/26, C 07 G 15/00**

(30) Priorität: 13.01.79 DE 2901297

(43) Veröffentlichungstag der Anmeldung:
23.07.80 Patentblatt 80/15

(84) Benannte Vertragsstaaten:
AT BE CH DE FR GB IT NL SE

(71) Anmelder: HOECHST AKTIENGESELLSCHAFT
Zentrale Patentabteilung Postfach 80 03 20
D-6230 Frankfurt/Main 80(DE)

(72) Erfinder: Geiger, Rolf, Dr.
Heinrich-Bleicher-Strasse 33
D-6000 Frankfurt am Main 50(DE)

(72) Erfinder: Regitz, Günter, Dr.
Dachbergstrasse 68
D-6232 Bad Soden am Taunus(DE)

(72) Erfinder: Geisen, Karl, Dr.
Jahnstrasse 43
D-6000 Frankfurt am Main(DE)

(72) Erfinder: Summ, Hans-Dieter, Dr.
Heimchenweg 48
D-6230 Frankfurt am Main 80(DE)

(72) Erfinder: Neubauer, Horst, Dr.
Am Eichkopf 12
D-6240 Königstein/Taunus(DE)

(54) Neue sulfonierte Insuline und Verfahren zu ihrer Herstellung.

(57) Gegenstand der Erfindung sind Insulinderivate der allgemeinen Formel I

$$\left.\begin{array}{l} \text{H-Gly-Ile-} \ \ldots\ldots \ \text{(A-Kette)} \\ \text{X-Phe-Val-} \ldots\ldots \ \text{(B-Kette)} \end{array}\right\} \text{Insulin} \qquad (I)$$

in der X einen eine Sulfosäuregruppe tragenden Alkanoyl- oder Cycloalkanoylrest mit 2 - 10 C-Atomen bedeutet, der ggf. auch Carbonamid-, Urethan-, Hydroxy- oder Carboxylgruppen enthält, sowie ein Verfahren zur Herstellung dieser Verbindungen durch Umsetzung von $N^{\alpha(A1)}, N^{\varepsilon \ (B29)}$-Ditert.-Butyloxycarbonylinsulin mit einem Überschuß an einem aktivierten Derivat einer Carbonsäure X-OH, und behandeln des Reaktionsprodukts mit einer starken Säure.

EP 0 013 568 A1

- 1 -

HOECHST AKTIENGESELLSCHAFT      HOE 79/F 006          Dr.LI/Lo

Neue sulfonierte Insuline und Verfahren zu ihrer Herstellung


Gegenstand der Erfindung sind neue Insulinderivate der
allgemeinen Formel I


$$\left.\begin{array}{l} \text{H-Gly-Ile-}\ldots\ldots \text{ (A-Kette)} \\ \text{X-Phe-Val-}\ldots\ldots \text{ (B-Kette)} \end{array}\right\} \text{Insulin (I)}$$


in der X einen eine Sulfosäuregruppe tragenden Alkanoyl- oder
Cycloalkanoylrest mit 2 - 10 C-Atomen bedeutet, der ggf.
auch Carbonamid-, Urethan, Hydroxy- oder Carboxylgruppen enthält.


Gegenstand der Erfindung ist ferner ein Verfahren zur Herstellung solcher Verbindungen der allgemeinen Formel I, in
der X die obige Bedeutung hat, das dadurch gekennzeichnet
ist, daß man $N^{\alpha (A1)}$, $N^{\varepsilon (B29)}$-Di-tert.-Butyloxycarbonyl-
insulin mit einem Überschuß an einem aktivierten Derivat einer Carbonsäure X-OH, wobei der Rest X die obige Bedeutung besitzt, umsetzt und das Reaktionsprodukt mit einer starken Säure behandelt.

Als Ausgangsprodukt für das N,N-Di-tert.-Butyloxycarbonylinsulin kommt Insulin verschiedener Spezies in Frage, bevorzugt verwendet man Rinder- oder Schweine-insulin oder ein Gemisch dieser beiden Insuline in beliebigem Mischungsverhältnis.

Bevorzugte aktivierte Derivate der Sulfocarbonsäuren X-OH sind z.B. gemischte oder symmetrische bzw. innere Anhydride. Ferner können aktive Ester mit Hydroxyverbindungen, die einen pK-Wert zwischen 4.0 und 8.0 besitzen, verwendet werden. Als solche Hydroxyverbindungen werden insbesondere die in der Peptidchemie üblichen Verbindungen, wie 4-Nitrophenol, 2.4-Dinitrophenol, 2.4.5-Trichlorphenol, Pentachlorphenol, Pentafluorphenol, N-Hydroxysuccinimid, N-Hydroxyphthalimid, 1-Hydroxy-benzotriazol oder 3-Hydroxy-4-oxo-3,4-dihydro-1.2.3-benzotriazin zur Herstellung der aktivierten Ester verwendet.

Man arbeitet vorteilhaft mit einem Überschuß bis zu 6 Äquivalenten, vorzugsweise mit 1,5 - 4 Äquivalenten an aktiviertem Säurederivat.

Als Sulfocarbonsäuren der Formel X-OH kommen z.B. folgende typischen Verbindungen infrage:

0013568

| Säure | bevorzugtes aktiviertes Derivat | |
|---|---|---|
| ß-Sulfo-propionsäure | akt. Ester, | cycl. Anhydrid |
| ß-Sulfo-hexancarbonsäure | akt. Ester, | gem. Anhydrid |
| $\gamma$-Sulfo-butancarbonsäure | akt. Ester, | gem. Anhydrid |
| Sulfo-bernsteinsäure | akt. Ester, | cycl. Anhydrid |
| Sulfo-äpfelsäure | akt. Ester | |
| N-Acetyl-cysteinsäure | akt. Ester | |
| N-Methyloxycarbonyl-cysteinsäure | akt. Ester | |
| N-Isobutyloxycarbonyl-cysteinsäure | akt. Ester | |
| N-Cyclohexyloxycarbonyl-cysteinsäure | akt. Ester | |

Gemischte Anhydride sind bevorzugt solche mit Kohlensäure-äthyl- oder isobutylester.

Bei der Umsetzung von $N^{\alpha(A1)}$, $N^{\varepsilon(B29)}$-Di-tert.-Butyl-oxycarbonylinsulin wird dessen noch freie Aminogruppe am Phenylalanin $^{B1}$ selektiv mit einem der erfindungsgemäßen aktivierten Carbonsäurederivate umgesetzt. Dabei ist es überraschend, daß trotz des verwendeten Überschusses an Carbonsäure-derivat keine anderen funktionellen Gruppen, die reichlich im Molekül vorhanden sind, angegriffen werden.

$N^{\alpha(A1)}$, $N^{\varepsilon(B29)}$-Di-tert.-Butyloxycarbonylinsulin ($BOC_2$-Insulin ist bekannt. Es wird beispielsweise nach der deutschen Patentschrift 1 793 517 hergestellt.

Zur Umsetzung mit den erfindungsgemäßen aktiven Carbonsäure-Derivaten löst man Di-Boc-Insulin vorteilhaft in der etwa 20- bis 50-fachen Menge etwa 60- bis 100-prozentigen Pyridins. Anstelle des Pyridins können auch ein N.N-Dialkylcarbonsäureamid oder Lösungsmittel wie Phosphor-säuretris-dimethylamid, Dimethylsulfoxyd oder Tetramethylharnstoff verwendet werden. Als Dialkylcarbonsäureamid werden beispielsweise verwendet: Dimethylformamid, Dimethylacetamid, Diäthylformamid oder N-Methyl-pyrrolidon.

Die aktiven Carbonsäure-Derivate der zugrunde liegenden Säure X-OH werden vorzugsweise dadurch hergestellt, daß man 1 Mol der Carbonsäure X-OH mit 1 Mol Dicyclohexylcarbodiimid und 1 - 1.2 Mol einer der aktive Ester bildenden Hydroxyverbindungen in einem der genannten Lösungsmittel umsetzt.

Nach beendeter Reaktion wird das erfindungsgemäße Insulinderivat mit Äther ausgefällt, 10 Min. bis 2 Stunden in der etwa 10-fachen Gewichtsmenge einer starken Säure, z.B. Trifluoressigsäure oder HCl in Eisessig aufbewahrt und wiederum mit Äther, Essigester oder Aceton ausgefällt.

Die Reaktionsprodukte werden nach 1 - 2-maliger Fällung z.B. durch Chromatographie an einem vernetzten Dextran-Gel (z.B. Sephadex G 50 der Firma Pharmacia, Uppsala) gereinigt. Vorteilhaft ist auch eine Ionenaustauscherreinigung an einem Gel desselben Typs, das saure oder basische Gruppen trägt (z.B. SE-, DEAE-, QAE- oder SP-Sephadex). Solche Reinigungen sind z.B. für Insulinderivate in Hoppe-Seyler's J. Phys. Chem. 353 (1972), Seiten 599 - 617 beschrieben.

Die neuen Insulin-Derivate besitzen volle oder nahezu volle Insulin-Aktivität, unterscheiden sich jedoch vom Insulin durch veränderte physikalische Eigenschaften, wie z.B. Erhöhung der Löslichkeit und Verschiebung des isoelektrischen Punktes.

0013568

Die erfindungsgemäßen Verbindungen zeigen ferner nur noch geringes Bindungsvermögen an Insulin-Antikörper und sind deshalb zur Behandlung von Diabetes Mellitus auch in Fällen geeignet, in denen hohe Antikörper-Titer sehr große Insulinmengen bei der Behandlung verlangen würden.

Diese Eigenschaft ist besonders bemerkenswert, weil die früher verwendeten, nicht selektiv sulfurierten Insuline, die ebenfalls geringere Antikörperbindung aufwiesen, nur noch einen Bruchteil der biologischen Wirkung des Ausgangsinsulins besaßen.

Die nachfolgenden Beispiele sollen die Erfindung näher erläutern. Zur Charakterisierung der acylierten Insuline dient die Papierelektrophorese (6 Stunden 200 Volt in Essigsäure/Ameisensäure pH 2. Anfärben mit Bromphenolblau). Die Wanderungsstrecke der erfindungsgemäßen Verbindungen entspricht der eines zweifach acylierten Insulins und ist für den Umsetzungsgrad beweisend. Sie kommt dadurch zustande, daß in diesen Verbindungen nicht nur eine Aminogruppe acyliert ist, sondern durch die vollständige Dissoziation der Sulfosäure bei pH 2 das Säureanion etwa denselben Bremseffekt ausübt wie die Acylierung einer Aminogruppe.

Beispiele

Beispiel 1

Allgemeine Vorschrift zur Herstellung der erfindungsgemäßen
Acylderivate des Insulins

a) 1 mMol X-OH und 1.2 mMol einer zur Aktivierung dienenden Hydroxyverbindungen wie 4-Nitrophenol, 2.4-Dinitrophenol, 2.4.5-Trichlorphenol, Pentachlorphenol, Pentafluorphenol, N-Hydroxysuccinimid, N-Hydroxyphthalimid,
1-Hydroxybenzotriazol oder 3-Hydroxy-4-oxo-3.4-dihydro-
1.2.3-benzotriazin werden in 3 ml Dimethylformamid gelöst.
Man kühlt auf 0°C und gibt 210 mg Dicyclohexylcarbodiimid zu, läßt langsam auf Raumtemperatur kommen und
rührt noch 3 Stunden bei Raumtemperatur weiter. Nach
Abfiltrieren des Harnstoffs wird die Lösung der aktivierten Säuren nach Beispiel b) direkt mit $Boc_2$-Insulin
umgesetzt.

b) 620 mg (0.1 mMol) $Boc_2$-Insulin, werden in 15 ml Dimethylacetamid gelöst und mit 0.6 ml (0.2 mMol) der
nach a) hergestellten Lösung eines aktivierten Derivates einer Säure X-OH versetzt. Nach 3 - 5 Stunden
Rühren bei Raumtemperatur fällt man das Reaktionsprodukt mit Äther oder Essigester aus.

c) Eine nach b) hergestellte Verbindung wird in 6 ml Trifluoressigsäure gelöst und 1 Stunde bei Raumtemperatur
aufbewahrt. Dann wird das auf diese Weise von Schutzgruppen befreite Reaktionsprodukt mit Äther gefällt.
Die Reinigung erfolgt z.B. durch Lösen in Wasser und
Chromatographie der Verbindung in einer mit Sephadex
(Fa. Pharmacia, Uppsala) G-50 gefüllten Säure 100 x 2.5 cm
mit 0.5 - 1 prozentiger Essigsäure als Lösungs- und
Elutionsmittel.

Beispiel 2

600 mg (0.1 mMol) Boc$_2$-Insulin werden in 10 ml Dimethylformamid gelöst. Man versetzt mit 55 mg ß-Sulfopropionsäure-anhydrid          und rührt 6 Stdn. bei Raumtemperatur. Nach etwa 30 Min. stellt man mit N-Ethylmorpholin auf ein scheinbares pH von etwa 7.5 ein.

Das Reaktionsprodukt wird mit Äther gefällt, nach Beispiel
1c von den Boc-Schutzgruppen befreit, aus der Trifluoressigsäurelösung wieder mit Äther gefällt und wie folgt
gereinigt:

Eine Säule 4 x 35 cm SP-Sephadex in einem aus 2.55 l
Wasser, 0.45 l Essigsäure, 2.0 l Isopropanol und 14.6 g
NaCl bestehenden Puffergemisch, das mit etwas konz.
Ammoniak auf pH 3 gestellt worden war, wird mit dem Rohprodukt, in 10 ml dieses Puffers gelöst, beschickt. Man
eluiert mit demselben Puffer und einem NaCl-Gradienten von
0.05 bis 0.25 M, wobei der vorgelegte 0.05 M NaCl Puffer
250 ml beträgt.

Man faßt Fraktionen entsprechend der UV-Extinktion zusammen.
Der erste hohe Peak entspricht der gesuchten Verbindung.
Man löst in wenig 0.1 %iger Ammonacetatlösung, lyophilisiert die vereinigten Fraktionen und entsalzt an einer
Säule "Biogel P 6" der Firma Bio-Rad (Biogel P 6 ist ein
vernetztes Polyacrylamid). Das salzfreie Eluans wird gefriergetrocknet. Ausb. 320 - 380 mg.

In der Papierelektrophorese bei pH 2 muß die Laufstrecke
der Verbindung der von Boc$_2$-Insulin entsprechen.

0013568

## Patentansprüche:

1. Insulinderivate der allgemeinen Formel I

$$\left.\begin{array}{l} \text{H-Gly-Ile-.....} \quad \text{(A-Kette)} \\ \text{X-Phe-Val-.....} \quad \text{(B-Kette)} \end{array}\right\} \text{Insulin} \qquad \text{(I)}$$

in der X einen eine Sulfosäuregruppe tragenden Alkanoyl- oder Cycloalkanoylrest mit 2 - 10 C-Atomen bedeutet, der ggf. auch Carbonamid-, Urethan, Hydroxy- oder Carboxylgruppen enthält.

2. Verfahren zur Herstellung von Verbindungen der allgemeinen Formel I, in der X die obige Bedeutung hat, das dadurch gekennzeichnet ist, daß man $N^{\alpha(A1)}$, $N^{\varepsilon(B29)}$-Di-tert.-Butyloxycarbonylinsulin mit einem Überschuß an einem aktivierten Derivat einer Carbonsäure X-OH, wobei der Rest X die obige Bedeutung besitzt, umsetzt und das Reaktionsprodukt mit einer starken Säure behandelt.

**Patentanspruch Österreich:**

Verfahren zur Herstellung von Insulinderivaten der allgemeinen Formel I

$$\left.\begin{array}{l} \text{H-Gly-Ile-..... (A-Kette)} \\ \text{X-Phe-Val-..... (B-Kette)} \end{array}\right\} \text{Insulin} \qquad \text{(I)}$$

in der X einen eine Sulfosäuregruppe tragende Alkanoyl- oder Cycloalkanoylrest mit 2 - 10 C-Atomen bedeutet, der ggf. auch Carbonamid-, Urethan-, Hydroxy- oder Carboxyl-gruppen enthält, dadurch gekennzeichnet, daß man $N^{\alpha}$ (A1), $N^{\epsilon}$ (B29) - Di-tert.-Butyloxycarbonylinsulin mit einem Überschuß an einem aktivierten Derivat einer Carbonsäure X-OH, wobei der Rest X die obige Bedeutung besitzt, umsetzt und das Reaktionsprodukt mit einer starken Säure behandelt.

# EUROPÄISCHER RECHERCHENBERICHT

Europäisches Patentamt

| EINSCHLÄGIGE DOKUMENTE | | KLASSIFIKATION DER ANMELDUNG (Int.Cl⁴) |
|---|---|---|
| **Kategorie** Kennzeichnung des Dokuments mit Angabe, soweit erforderlich, der maßgeblichen Teile | **betrifft Anspruch** | C 07 C 103/52<br>A 61 K 37/26<br>C 07 G 15/00 |
| CHEMICAL ABSTRACTS, Band 84, Nr. 1, 5. Januar 1976, Seite 181, Nr. 1891n<br>Columbus, Ohio, U.S.A.<br>R. OBERMEIER et al.: "New bifunctional reagent for the intramolecular crosslinking of insulin"<br>& HOPPE-SEYLER'S Z. PHYSIOL. CHEM. 1975, 356(10), 1631-4<br>  * Zusammenfassung *<br><br>-- | 1,2 | |
| FR - A - 2 283 124 (HOECHST)<br>  * Insgesamt *<br><br>-- | 1 | **RECHERCHIERTE SACHGEBIETE (Int. Cl³)**<br><br>C 07 C 103/52<br>A 61 K 37/26<br>C 07 G 15/00 |
| FR - A - 2 100 978 (HOECHST)<br>  * Insgesamt *<br><br>-- | 1,2 | |
| CH - A - 602 599 (CIBA-GEIGY)<br>  * Spalte 3, Zeilen 37-56 *<br><br>-- | 1,2 | |
| BIOCHEM. J., Nr. 121, 1971, Seiten 737-745<br>Printed in Great Britain<br>D.G. LINDSAY et al.: "The Acetylation of Insulin"<br>  * Insgesamt *<br><br>---- | 1 | **KATEGORIE DER GENANNTEN DOKUMENTE**<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |

Der vorliegende Recherchenbericht wurde für alle Patentansprüche erstellt.

| Recherchenort | Abschlußdatum der Recherche | Prüfer |
|---|---|---|
| Den Haag | 14-04-1980 | RAJIC |

EPA form 1503.1  06.78